# EUROPÄISCHE PATENTSCHRIFT

(11) **EP 2 786 743 B1**
(45) Veröffentlichungstag und Bekanntmachung des Hinweises auf die Patenterteilung: **22.05.2019**
(21) Anmeldenummer: 14158263.5
(22) Anmeldetag: 07.03.2014
(51) Int. Cl.: A61K 8/60, A61Q 19/00, A61Q 19/10

(54) **Mischungszusammensetzung enthaltend Rhamnolipide**
Mixture composition containing rhamnolipids
Composition de mélange contenant des rhamnolipides

(30) Priorität: 02.04.2013 DE 102013205756
(43) Veröffentlichungstag der Anmeldung: 08.10.2014
(73) Patentinhaber: Evonik Degussa GmbH, 45128 Essen (DE)
(72) Erfinder: Schilling, Martin, 53121 Bonn (DE); Hartung, Christian, 45133 Essen (DE); Cabirol, Fabien, 507740 Singapore (SG); Schaffer, Steffen, 45699 Herten (DE); Allef, Petra, 45128 Essen (DE)
(74) Vertreter: Evonik Patent Association

(56) Entgegenhaltungen:
- EP-A1- 1 445 302
- EP-A1- 2 410 039
- EP-A1- 2 735 605
- EP-A2- 0 153 634
- WO-A1-01/10447
- WO-A1-2012/010406
- WO-A2-93/14767
- ORTIZ A ET AL: "Effects of dirhamnolipid on the structural properties of phosphatidylcholine membranes", INTERNATIONAL JOURNAL OF PHARMACEUTICS, ELSEVIER BV, NL, Bd. 325, Nr. 1-2, 15. November 2006 (2006-11-15), Seiten 99-107, XP027972535, ISSN: 0378-5173 [gefunden am 2006-11-15]
- ALEJANDRO P. ROONEY ET AL: "Isolation and characterization of rhamnolipid-producing bacterial strains from a biodiesel facility", FEMS MICROBIOLOGY LETTERS, Bd. 295, Nr. 1, 1. Juni 2009 (2009-06-01), Seiten 82-87, XP055103888, ISSN: 0378-1097, DOI: 10.1111/j.1574-6968.2009.01581.x
- M. L. CHEN ET AL: "Solution Self-Assembly and Adsorption at the Air-Water Interface of the Monorhamnose and Dirhamnose Rhamnolipids and Their Mixtures", LANGMUIR, Bd. 26, Nr. 23, 7. Dezember 2010 (2010-12-07), Seiten 18281-18292, XP055132334, ISSN: 0743-7463, DOI: 10.1021/la1031812
- OCHSNER: "Production of Pseudomonas aeruginosa rhamnolipid biosurfactants in heterologous hosts", APPLIED AND ENVIRONMENTAL MICROBIOLOGY, Bd. 61, Nr. 9, 1. Januar 1995 (1995-01-01) , Seite 3503, XP055005925, ISSN: 0099-2240

## Beschreibung

### Gebiet der Erfindung

Gegenstand der Erfindung ist eine Mischungszusammensetzung enthaltend Rhamnolipide, ein Verfahren zu ihrer Herstellung und ihre Verwendung zur Herstellung von Formulierungen.

### Stand der Technik

Rhamnolipide sind Glykolipide, die in freier Natur von bestimmten Bakterien, beispielsweise *Pseudomonas aeruginosa*, produziert werden. Üblicherweise produzieren die Mikroorganismen Mischungen von Rhamnolipiden enthaltend mono- und di-Rhamnolipide, die eine beziehungsweise zwei Rhamnoseeinheiten pro Molekül aufweisen und unterschiedlich lange Lipidketten enthalten können.

EP153634 beschreibt Mischungszusammensetzung mit einem fast ausgeglichenem Gewichtsverhältnis von mono- zu di-Rhamnolipiden.

Ebenso beschreibt die EP0499434 in Beispiel 3 Rhamnolipid Zusammensetzungen mit ausgeglichenem Gewichtsverhältnis der beiden Komponenten.

EP2410039 beschreibt Reinigungsmittel enthaltend mono- und di-Rhamnolipide mit einem Gewichtsverhältnis von 95:5 bis 45:55.

Die Charakterisierung der Oberflächenaktivitäten von reinen di-Rhamnolipiden, reinen mono-Rhamnolipiden und deren Mischungen werden beispielsweise in Chen et al., Solution self-assembly and adsorption at the air-water interface of the monorhamnose and dirhamnose rhamnolipids and their mixtures, Langmuir. 2010 Dec 7;26(23):18281-92 beschrieben.

Die WO93/14767 offenbart eine pharmazeutische Zubereitung, enthaltend als Wirkstoff mindestens ein Rhamnolipid oder ein pharmazeutisch verträgliches Salz davon und einen Träger und / oder ein Verdünnungsmittel.

Ein Nachteil der bisher bekannten, durch einfache, fermentative Verfahren herstellbaren Rhamnolipide ist deren relativ geringer Anteil an di-Rhamnolipiden. Diese Rhamnolipid-Zusammensetzungen enthalten über dies hinaus relativ häufig als unerwünschte Nebenprodukte Rhamnolipide mit nur einer Acyl-Kette.

Durch Fraktionierung aufgereinigte, reine di-Rhamnolipid-Zusammensetzungen zeichnen sich bei der Verwendung als Kosmetikum durch ein stumpfes, austrocknendes Hautgefühl aus.

Aufgabe der Erfindung war es, Rhamnolipid Kompositionen bereitzustellen, die über gute Schaumeigenschaften verfügen und überdies hinaus ein leichtes Hautgefühl bei der Verwendung in Hautpflege- oder -reinigungsmitteln aufweisen.

### Beschreibung der Erfindung

Überraschenderweise wurde gefunden, dass die im Folgenden beschriebene Mischungszusammensetzung die der Erfindung gestellte Aufgabe zu lösen vermag.

Gegenstand der vorliegenden Erfindung sind daher Mischungszusammensetzungen enthaltend bestimmte Rhamnolipide in definierten Gewichtsverhältnissen.

Ein weiterer Gegenstand der Erfindung ist ein Verfahren zur Herstellung der erfindungsgemäßen Mischungszusammensetzungen mit gentechnisch veränderten Zellen.

Ein Vorteil der erfindungsgemäßen Mischungszusammensetzungen ist deren überragende Schaumstabilität im Wässrigen.

Noch ein Vorteil der erfindungsgemäßen Mischungszusammensetzungen ist deren überragendes Schaumvolumen im Wässrigen.

Ein weiterer Vorteil der erfindungsgemäßen Mischungszusammensetzungen ist deren hervorragendes Anschäumverhalten.

Noch ein Vorteil der erfindungsgemäßen Mischungszusammensetzungen ist deren einfache Formulierbarkeit in beliebigen wässrigen, tensidischen Systemen.

Ein weiterer Vorteil der erfindungsgemäßen Mischungszusammensetzungen ist deren gute Verdickbarkeit mit konventionellen Verdickern in Formulierungen.

Noch ein Vorteil ist ihre gute Abwaschbarkeit von Haut und Haaren.

Ein weiterer Vorteil der erfindungsgemäßen Mischungszusammensetzungen ist ihre Mildheit bzw. gute physiologische Verträglichkeit, insbesondere charakterisiert durch einen hohen Wert im Red Blood-Cell (RBC) Test.

Noch ein Vorteil ist deren gutes Hautgefühl während und nach dem Waschen.

Ein weiterer Vorteil der erfindungsgemäßen Mischungszusammensetzungen ist, dass sie ein weiches Hautgefühl nach dem Waschen hinterlassen.

Noch ein Vorteil der erfindungsgemäßen Mischungszusammensetzungen ist, dass sie ein glattes Hautgefühl nach dem Waschen hinterlassen.

Ein weiterer Vorteil der erfindungsgemäßen Mischungszusammensetzungen ist, dass sie auf der Haut einen rückfettenden Effekt aufweisen.

Ein weiterer Vorteil der erfindungsgemäßen Mischungszusammensetzungen ist, dass sie sich im Wesentlichen frei von Öl darstellen lassen.

Unter dem Begriff "Rhamnolipid" im Zusammenhang mit der vorliegenden Erfindung werden insbesondere Verbindungen der allgemeinen Formel (I) oder deren Salze verstanden, 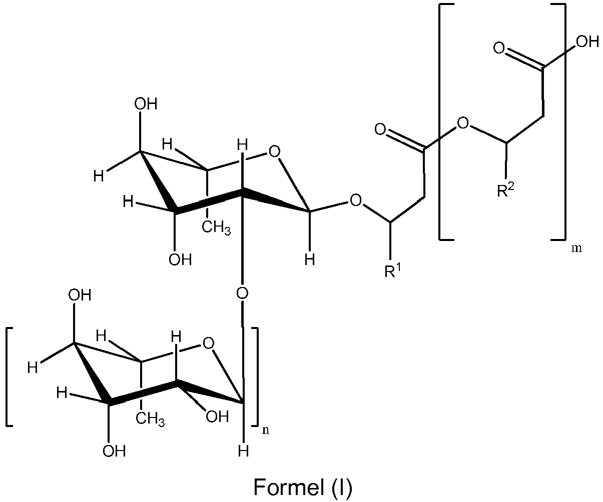 wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.

Unter dem Begriff "di-Rhamnolipid" im Zusammenhang mit der vorliegenden Erfindung werden Verbindungen der allgemeinen Formel (I) oder deren Salze verstanden, bei denen n =1.

Unter dem Begriff "mono-Rhamnolipid" im Zusammenhang mit der vorliegenden Erfindung werden Verbindungen der allgemeinen Formel (I) oder deren Salze verstanden, bei denen n =0.

Distinkte Rhamnolipide werden gemäß folgender Nomenklatur abgekürzt:
Unter "diRL-CXCY" werden di-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Unter "monoRL-CXCY" werden mono-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX". Für Rhamnolipide mit m=0 wird entsprechend monoRL-CX bzw. diRL-CX verwendet. Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.

Der "pH-Wert" im Zusammenhang mit der vorliegenden Erfindung ist definiert als der Wert, welcher für entsprechenden Stoff bei 25 °C nach fünf Minuten Rühren mit einer gemäß ISO 4319 (1977) kalibrierten pH-Elektrode gemessen wird.

Unter dem Begriff "wässriges Medium" im Zusammenhang mit der vorliegenden Erfindung ist eine Zusammensetzung zu verstehen, die mindestens 5 Gew.-% Wasser, bezogen auf die betrachtete Gesamtzusammensetzung enthält.

Alle angegebenen Prozent (%) sind, wenn nicht anders angegeben, Massenprozent.

Gegenstand der vorliegenden Erfindung ist eine Mischungszusammensetzung enthaltend Rhamnolipide, dadurch gekennzeichnet, dass die Mischungszusammensetzung
51 Gew.-% bis 95 Gew.-%, bevorzugt 70 Gew.-% bis 90 Gew.-%, besonders bevorzugt 75 Gew.-% bis 85 Gew.-%, diRL-C10C10 und
0,5 Gew.-% bis 9 Gew.-%, bevorzugt 0,5 Gew.-% bis 3 Gew.-%, besonders bevorzugt 0,5 Gew.-% bis 2 Gew.-%, monoRL-C10C10
enthält,
wobei sich die Gewichtsprozente auf die Summe aller enthaltenen Rhamnolipide beziehen,
mit der Maßgabe, dass das Gewichtsverhältnis von di-Rhamnolipiden zu mono-Rhamnolipiden größer 91:9, bevorzugt größer 97:3, besonders bevorzugt größer 98:2 ist.

Eine bevorzugte erfindungsgemäße Mischungszusammensetzung ist dadurch gekennzeichnet, dass die Mischungszusammensetzung
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1
enthält,
wobei sich die Gewichtsprozente auf die Summe aller enthaltenen Rhamnolipide beziehen.

Eine weitere bevorzugte erfindungsgemäße Mischungszusammensetzung ist dadurch gekennzeichnet, dass die Mischungszusammensetzung
0,5 bis 25 Gew.-%, bevorzugt 5 Gew.-% bis 15 Gew.-%, besonders bevorzugt 7 Gew.-% bis 12 Gew.-%, diRL-C10C12
enthält,
wobei sich die Gewichtsprozente auf die Summe aller enthaltenen Rhamnolipide beziehen.

Es ist weiterhin bevorzugt, wenn die erfindungsgemäße Mischungszusammensetzung 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und/oder, bevorzugt und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthält,
wobei sich die Gewichtsprozente auf die Summe aller enthaltenen Rhamnolipide beziehen.

Es kann vorteilhaft sein und ist daher bevorzugt, wenn die erfindungsgemäße Mischungszusammensetzung
0,1 Gew.-% bis 25 Gew.-%, bevorzugt 2 Gew.-% bis 10 Gew.-%, besonders bevorzugt 4 Gew.-% bis 8 Gew.-%, diRL-C8C10,
enthält,
wobei sich die Gewichtsprozente auf die Summe aller enthaltenen Rhamnolipide beziehen.

Eine besonders bevorzugte erfindungsgemäße Mischungszusammensetzung ist dadurch gekennzeichnet, dass die Mischungszusammensetzung
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1,
0,5 bis 25 Gew.-%, bevorzugt 5 Gew.-% bis 15 Gew.-%, besonders bevorzugt 7 Gew.-% bis 12 Gew.-%, diRL-C10C12,
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthält,
wobei sich die Gewichtsprozente auf die Summe aller enthaltenen Rhamnolipide beziehen.

Es ist überdies hinaus bevorzugt, wenn die erfindungsgemäße Mischungszusammensetzung Rhamnolipide der Formel monoRL-CX bzw. diRL-CX in nur kleinen Mengen enthält. Insbesondere enthält die erfindungsgemäße Mischungszusammensetzung bevorzugt
0 Gew.-% bis 5 Gew.-%, bevorzugt 0 Gew.-% bis 3 Gew.-%, besonders bevorzugt 0 Gew.-% bis 1 Gew.-%, diRLC10, wobei sich die Gewichtsprozente auf die Summe aller enthaltenen Rhamnolipide beziehen, und unter dem Begriff "0 Gew.-%" keine nachweisbare Menge zu verstehen ist.

Die erfindungsgemäße Mischungszusammensetzung enthält bevorzugt mindestens 60 Gew.-%, bevorzugt mindestens 80 Gew.-%, besonders bevorzugt mindestens 90 Gew.-%, insbesondere mindestens 95 Gew.-%, Rhamnolipide, wobei sich die Gewichtsprozente auf die Trockensubstanz der gesamten Mischungszusammensetzung beziehen.
Es ist erfindungsgemäß bevorzugt, dass die erfindungsgemäßen Mischungszusammensetzungen im Wesentlichen frei von fettem Öl (bei 20 °C flüssigen Acylglycerolen) sind und somit insbesondere weniger als 0,5 Gew.-%, insbesondere weniger als 0,1 Gew.-%, besonders bevorzugt keine nachweisbaren Mengen, fettes Öl bezogen auf die gesamte Mischungszusammensetzung enthalten. Die erfindungsgemäßen Mischungszusammensetzungen lassen sich durch Abmischen der Reinsubstanzen herstellen, wobei die Reinsubstanzen aus herkömmlich hergestellten Rhamnolipid-Mischungen aufgereinigt werden können. Entsprechende Aufreinigungsverfahren sind beispielsweise selektive Kristallisationen und chromatographische Methoden. Entsprechende Verfahren sind in Heyd et al., Development and trends of biosurfactant analysis and purification using rhamnolipids as an example, Anal Bioanal Chem. 2008 Jul;391(5):1579-90 beschrieben.

Insbesondere die im Folgenden beschriebenen Verfahren, welche ebenfalls Gegenstand der vorliegenden Erfindung sind, eignen sich zur Herstellung von den erfindungsgemäßen Mischungszusammensetzungen. Das erfindungsgemäße Verfahren umfasst die Verfahrensschritte:
Ia) Bereitstellen einer *Pseudomonas putida* Zelle, die derart gentechnisch verändert wurde, dass sie jeweils mindestens ein Gen der Gruppe rhIA, rhIB und rhIC überexprimiert, wobei die Gene ausgewählt sind aus denen aus *P*. *aeruginosa*,
IIa) in Kontakt Bringen der erfindungsgemäßen Zelle mit einem Medium beinhaltend mindestens eine Kohlenstoffquelle,
IIIa) Kultivieren der Zelle unter Bedingungen, die es der Zelle ermöglichen, aus der Kohlenstoffquelle Rhamnolipid zu bilden und
IVa) gegebenenfalls Isolierung der gebildeten Rhamnolipide,
dadurch gekennzeichnet, dass das Gen rhIC verglichen zu rhIB stärker, insbesondere mindestens 1,5-fach, bevorzugt mindestens 2-fach, besonders bevorzugt mindestens 10-fach stärker, überexprimiert wird.

Die relative Stärke der oben beschriebenen Überexpression lässt sich beispielsweise mit Hilfe von RT-PCR bestimmen, bei der die Menge an gebildeter mRNA für das jeweilige Gen bestimmt wird.
Eine Regulierung der Stärke der Expression kann der Fachmann gezielt beispielsweise durch die Wahl von Promotoren oder durch den Einsatz induzierbarer Promotoren in Kombination mit Menge an Induktor erreichen oder auch durch Genvervielfachungen.

Ein ebenso bevorzugtes, alternatives erfindungsgemäße Verfahren umfasst die Verfahrensschritte:
Ib) Bereitstellen einer *Pseudomonas putida* Zelle, die derart gentechnisch verändert wurde, dass sie jeweils mindestens ein exogenes Gen der Gruppe rhIA, rhIB und rhIC, von denen mindestens eines unter der Kontrolle eines induzierbaren Promotors steht, aufweist,
IIb) in Kontakt Bringen und Kultivieren der erfindungsgemäßen Zelle mit einem Medium beinhaltend mindestens eine Kohlenstoffquelle unter Erreichung einer Zelldichte von 1-30 g Zelltrockenmasse pro L Fermentationsbrühe, bevorzugt 2-20 g Zelltrockenmasse pro L Fermentationsbrühe, besonders bevorzugt 5-15 g Zelltrockenmasse pro L Fermentationsbrühe,
IIIb) Induzieren des mindestens einen induzierbaren Promotors und Kultivieren der Zelle unter Bedingungen, die es der Zelle ermöglichen, aus der Kohlenstoffquelle Rhamnolipid zu bilden und
IVb) gegebenenfalls Isolierung der gebildeten Rhamnolipide.

Unter dem Begriff "induzierbarer Promotor" ist im Zusammenhang mit der vorliegenden Erfindung ein Promotor zu verstehen, der durch eine Veränderung des die Zelle umgebenden Mediums seine Aktivität ändert. Veränderungen können zum Beispiel Temperaturänderungen und Konzentrationsänderungen von bestimmten Substanzen umfassen.
Unter dem Begriff "Induzieren des mindestens einen induzierbaren Promotors" ist im Zusammenhang mit der vorliegenden Erfindung zu verstehen, dass die Aktivität des induzierbaren Promotors durch eine Veränderung des die Zelle umgebenden Mediums erhöht wird.
Geeignete induzierbare Promotoren im Zusammenhang mit der vorliegenden Erfindung sind beispielsweise Promotoren, die durch Zugabe chemischer Induktoren (etwa Laktose, IPTG, Dicyclopropylketon, Tetracyclin, Doxycyclin, Propionat, Cumat, Benzoat, Arabinose, Rhamnose, Nikotinsäure, etc.) induziert werden, die durch veränderte Umweltbedingungen (etwa auftretender Phosphat- oder Schwefelmangel, veränderte Temperaturen oder pH, etc.) induziert werden, oder die durch bestimmte physiologische Zustände (etwa bestimmte Zelldichten oder Wachstumsraten bzw. - phasen) induziert werden.

Insbesondere bevorzugt in dem Verfahren eingesetzte induzierbare Promotoren sind ausgewählt aus der Gruppe der durch Dicyclopropylketon, Tetracyclin, Doxycyclin, Propionat, Cumat, Benzoat, Phosphatmangel, Schwefelmangel oder eine verminderte Wachstumsrate induzierbaren Promotoren.

Die Gene rhIA, rhIB und rhIC sind in beiden erfindungsgemäßen Verfahren bevorzugt ausgewählt aus denen aus *P*. *aeruginosa.*
Die beiden erfindungsgemäßen Verfahren werden bevorzugt nicht als Biotransformation durchgeführt; das heißt, dass die Rhamnolipide nicht aus Fettsäuren bzw. fettsäurehaltigen Verbindungen wie beispielsweise Fetten und Ölen gebildet werden, die von außen dem Verfahren zugeführt werden, sondern dass unter den in den Verfahren genannten Kohlenstoffquellen insbesondere solche verstanden werden, die überwiegend mindestens eine andere kohlestoffhaltige Verbindung als Fettsäuren bzw. fettsäurehaltigen Verbindungen enthalten.

Die erfindungsgemäßen Mischungszusammensetzungen lassen sich vorteilhaft in insbesondere kosmetischen Formulierungen einarbeiten.
Somit ist ein weiterer Gegenstand der vorliegenden Erfindung die Verwendung der erfindungsgemäßen Mischungszusammensetzungen zur Herstellung von Formulierungen, insbesondere von kosmetischen Formulierungen

Die Formulierungen enthalten bevorzugt neben den erfindungsgemäßen Mischungszusammensetzungen mindestens ein weiteres Tensid, wobei beispielsweise anionische, nichtionische, kationische und/oder amphotere Tenside eingesetzt werden können. Bevorzugt sind aus anwendungstechnischer Sicht Mischungen aus anionischen und nichtionischen Tensiden. Der Gesamttensidgehalt der wässrigen Formulierung beträgt vorzugsweise 5 bis 60 Gew.-% und besonders bevorzugt 15 bis 40 Gew.-%, bezogen auf die gesamte Formulierung.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-C14-Alkohole mit 3 EO, 4 EO oder 7 EO, C9-C11-Alkohol mit 7 EO, C13-C15- Alkohole mit 3 EO, 5 EO, 7 EO oder 8 EO, C12-C18-Alkohole mit 3 EO, 5 EO oder 7 EO und Mischungen aus diesen, wie Mischungen aus C12-C14-Alkohol mit 3 EO und C12-C18-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO(Propylenoxid)-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere.

Selbstverständlich sind auch gemischt alkoxylierte nichtionische Tenside einsetzbar, in denen EO-und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside eingesetzt werden.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsaurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide; bei den Polyhydroxyfettsäureamiden handelt es sich um Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Als anionische Tenside werden beispielsweise solche vom Typ der Sulfonate und Sulfate eingesetzt. Als Tenside vom Sulfonat-Typ kommen dabei vorzugsweise C9-C13-Alkylbenzolsulfonate, Olefinsulfonate, d.h. Gemische aus Alken- und Hydroxyalkansulfonaten sowie Disulfonaten, wie man sie beispielsweise aus C12-C18-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C12-C18-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C12-C18-Fettalkohole, beispielsweise aus Kokosfettalkohol, Talgfettalkohol, Lauryl-, Myristyl-, Cetyl- oder Stearylalkohol oder der C10-C20-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C12-C16-Alkylsulfate und C12-C18-Alkylsulfate sowie C14-C18-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN® erhalten werden können, sind geeignete anionische Tenside.

Auch die Schwefelsäuremonoester der mit 1 bis 6 Mol Ethylenoxid ethoxylierten geradkettigen oder verzweigten C7-C20-Alkohole, wie 2-Methyl-verzweigte C9-C11-Alkohole mit im Durchschnitt 3,5 Mol Ethylenoxid (EO) oder C12-C18-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C8-C18-Fettalkoholreste oder Mischungen aus diesen. Insbesondere bevorzugte Sulfosuccinate enthalten einen Fettalkoholrest, der sich von ethoxylierten Fettalkoholen ableitet. Dabei sind wiederum Sulfosuccinate, deren Fettalkohol-Reste sich von ethoxylierten Fettalkoholen mit enger Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen. Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Als amhpotere Tenside können solche oberflächenaktiven Verbindungen eingesetzt werden, die im Molekül mindestens eine quartäre Ammoniumgruppe und mindestens eine -COO⁻- oder -SO₃⁻ -Gruppe tragen. Besonders bevorzugte amphotere Tenside sind in diesem Zusammenhang Betain-Tenside wie Alkyl- oder Alkylamidopropylbetaine. Insbesondere sind hier Betaine wie die N-Alkyl-N,N-dimethylammonium-glycinate, z. B. das Kokosalkyl-dimethylammoniumglycinat, N-Acyl-aminopropyl-N,N-dimethylammoniumglycinate, z. B. das Kokosacylaminopropyldimethylammoniumglycinat, das C12-C18-Alkyl-dimethyl-acetobetain, das Kokosamidopropyl-dimethyl-acetobetain, 2-Alkyl-3-carboxymethyl-3-hydroxyethyl-imidazoline und Sulfobetaine mit jeweils 8 bis 18 C-Atomen in der Alkyl- oder Acylgruppe sowie das Kokosacylaminoethylhydroxyethylcarboxymethylglycinat bevorzugt. Ein besonders bevorzugtes zwitterionisches Tensid ist das unter der INCI-Bezeichnung Cocamidopropyl Betaine bekannte N,N-Dimethyl-N-(lauroylamidopropyl)ammoniumacetobetain.

Weitere geeignete amphotere Tenside bildet die Gruppe der Amphoacetate und Amphodiacetate, insbesondere beispielsweise Kokos- oder Laurylamphoacetate oder - diacetate, die Gruppe der Amphopropionate und Amphodipropionate sowie die Gruppe der aminosäurebasierten Tenside wie Acylglutamate, insbesondere Disodium Cocoyl Glutamate und Sodium Cocoyl Glutamate, Acylglycinate, insbesondere Cocoyl Glycinate, und Acylsarcosinate, insbesondere Ammonium Lauroyl Sarcosinate und Sodium Cocoyl Sarcosinate.

Die Formulierungen können des Weiteren mindestens eine zusätzliche Komponente enthalten, ausgewählt aus der Gruppe der
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Geruchsabsorber,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Die erfindungsgemäßen Mischungszusammensetzungen lassen sich vorteilhaft zur Reinigung von Oberflächen verwenden. Bei dieser Form der erfindungsgemäßen Verwendung ist die Oberfläche bevorzugt die Oberfläche eines Lebewesens, insbesondere eines Menschen, wobei solche Oberflächen besonders bevorzugt ausgewählt sind aus Haut und Haar.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beschreibung:

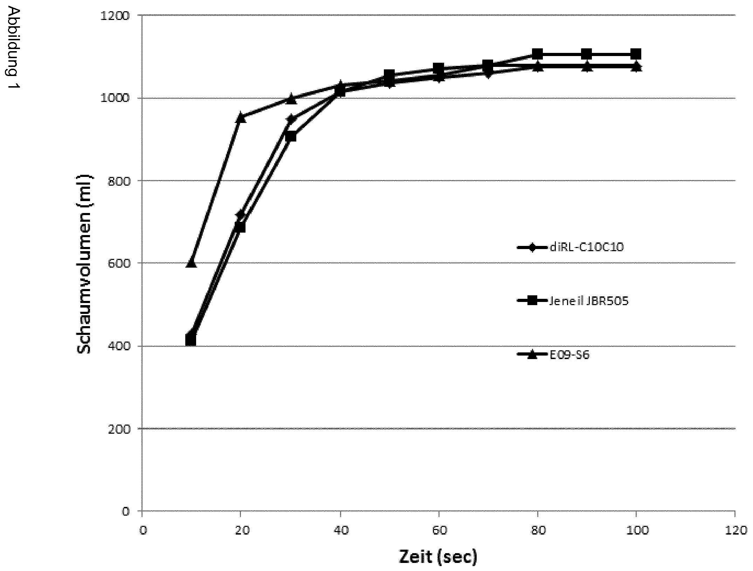

Abbildung 1: Anschäumverhalten gezeigt mit SITA-Messung

### Beispiele:

### Beispiel 1: Herstellung von diRL-C10C10 und monoRL-C10-C10

Die Herstellung der verschiedenen, reinen RL Formen erfolgt mittels präparativer Säulenchromatographie. Hierfür werden 750 g eines Silica 60 Gels (200 - 500 µm; 35 - 70 mesh; Sigma-Aldrich, Germany) in wassergesättigtem Ethylacetat (angesäuert mit 1 Gew. % Essigsäure) suspendiert und in eine Säule gefüllt (Durchmesser = 65mm, maximale Füllhöhe = 600mm, 1 l Lösungsmittelreservoir). 2 - 3 cm säurebehandelter Seesand (Riedel de Haen, Seelze, Germany) werden als Schutzschicht über die stationäre Phase geschichtet. Als Laufmittel wird ebenfalls wassergesättigtes Ethylacetat verwendet, das 1 Gew. % Essigsäure enthält. Eine kommerziell erhältliche RL Mischung (JBR 505, Jeneil Biosurfactants, ∼ 5 Gew. % Gesamtrhamnolipidkonzentration) wird gefriergetrocknet. 10 g der gefriergetrockneten RL Mischung werden in 5 Gew. % Konzentration im Laufmittel gelöst. Die Lösung wird auf die vorbereitete Säule gegeben. Der Laufmittelfluss wird auf 15 ml/min eingestellt. Das Eluat wird in 100 ml Fraktionen gesammelt und mittels Dünnschichtchromatographie und HPLC analysiert. Auf diese Weise können die verschiedenen RL Formen getrennt werden. Fraktionen gleicher Zusammensetzung werden vereinigt und das Lösungsmittel im Rotationsverdampfer abgezogen. Anschließend wird der Rückstand in Wasser gelöst, gefriergetrocknet und in Pulverform für die Anwendungstests verwendet. Um ausreichende Mengen zu erhalten, wird diese Prozedur mehrmals durchgeführt. Die Reinheit der erhaltenen Fraktionen wird mittels ¹H-NMR und HPLC auf > 99 Gew. % bestimmt.

### Beispiel 2: Herstellung einer Abmischung von diRL-C10C10 und monoRL-C10C10

Die in Beispiel 1 beschriebenen, reinen RL Formen werden in Pulverform in einem Verhältnis von diRL-C10C10 zu monoRL-C10C10 von 97,5:2,5 gemischt.

### Beispiel 3: Herstellung von Rhamnolipiden mit rhIABC aus P. aeruginosa PAO1 in P. putida, wobei die Expression des für die Rhamnosyltransferase RhIC kodierenden Gens vielfach stärker ist, als die des für die Rhamnosyltransferase RhlB kodierenden Gens rhIB

Um Rhamnolipide mit *rhIABC* aus *P. aeruginosa* PAO1 in einem *P. putida*-Stamm herzustellen, in dem die Expression des für die Rhamnosyltransferase RhIC kodierenden Gens vielfach stärker erfolgt, als die des für die Rhamnosyltransferase RhlB kodierenden Gens rhIB, wird das Plasmid pBBR1MCS2-Plac-rhIABC-T-Ptac-rhIC-T (Seq ID Nr. 1) konstruiert. Dazu wurden folgende DNA-Fragmente synthetisiert: *P. aeruginosa* PAO1-Gene *rhIA*, *rhIB* und *rhIC*, gefolgt von einem Terminator, gefolgt vom synthetischen *tac*-Promoter, gefolgt vom *P. aeruginosa* PAO1-Gene *rhIC* und einem Terminator, flankiert von einer *Hin*dIII- (5'-Ende) bzw. *Bsu*36I-Schnittstelle (3'-Ende) (Seq ID Nr. 2).
Die vom DNA-Synthese-Provider gelieferten Vektoren, die das synthetisierte DNA Fragment enthalten, werden mit *Hin*dIII und *Bsu*36I geschnitten und in den ebenfalls mit *Hin*dIII und *Bsu*36I geschnittenen Vektor pBBR1MCS-2 (Seq ID 3) mittels Fast Link Ligation Kit (Epicentre Technologies; Madison, WI, USA) ligiert. Der erhaltene Zielvektor pBBR1MCS2-Plac-rhIABC-T-Ptac-rhIC-T (pBBR1MCS-2 mit synthetisiertem Fragment Seq ID Nr. 2) hat eine Größe von 9336 Basenpaaren.
Die Transformation von *Pseudomonas putida* KT2440 mit dem Vektor pBBR1MCS2-Plac-rhIABC-T-Ptac-rhIC-T (Seq ID Nr. 1) erfolgt wie vorbeschrieben (Iwasaki et al. Biosci. Biotech. Biochem. 1994. 58(5):851-854). Die Plasmid-DNA von je 10 Klonen wird isoliert und analysiert. Der erhaltene, das Plasmid tragende Stamm wird *P. putida* KT2440 pBBR1MCS2-Plac-rhIABC-T-Ptac-rhIC-T genannt.

Der rekombinante Stamm *P. putida KT2440* pBBR1MCS2-Plac-rhIABC-T-Ptac-rhIC-T wird auf LB-Agar-Kanamycin (50 µg/ml)-Platten kultiviert.
Zur Produktion der Rhamnolipide wird das im Folgenden als M9-Medium bezeichnete Medium verwendet. Dieses Medium besteht aus 2 % (w/v) Glukose, 0,3 % (w/v) KH₂PO₄, 0,679 % Na₂HPO₄, 0,05 % (w/v) NaCl, 0,2 % (w/v) NH₄Cl, 0,049 % (w/v) MgSO₄ x 7 H₂O und 0,1 % (v/v) einer Spurenelementlösung. Diese besteht aus 1,78 % (w/v) FeSO₄ x 7 H₂O, 0,191 % (w/v) MnCl₂ x 7 H₂O, 3,65 % (w/v) HCl, 0,187 % (w/v) ZnSO₄ x 7 H₂O, 0,084 % (v/v) Na-EDTA x 2 H₂O, 0,03 % (v/v) H₃BO₃, 0,025 % (w/v) Na₂MoO₄ x 2 H₂O und 0,47 % (w/v) CaCl₂ x 2 H₂O. Der pH-Wert des Mediums wird mit NH₄OH auf 7,4 eingestellt und das Medium folgend mittels Autoklav (121 °C, 20 min) sterilisiert. Ein Einstellen des pH-Wertes während der Kultivierung ist nicht nötig.

Zur Untersuchung der Rhamnolipidproduktion im Schüttelkolben wird zunächst eine Vorkultur angesetzt. Hierzu wird eine Kolonie eines frisch auf LB-Agar-Platte ausgestrichenen Stammes verwendet und 10 ml LB-Medium in einem 100 ml Erlenmeyerkolben beimpft. Alle rekombinanten *P. putida-Stämme* werden im LB-Medium, dem 50 µg/ml Kanamycin zugesetzt wird, kultiviert. Die Kultivierung der *P*. putida-Stämme erfolgt bei 30 °C und 200 rpm über Nacht.
Die Vorkulturen werden verwendet, um 50 ml M9-Medium (+ 50 µg/ml Kanamycin) im 250 ml Erlenmeyerkolben zu beimpfen (Start-OD₆₀₀ 0,1). Die Kulturen werden bei 200 rpm und 30 °C kultiviert. Nach 24 h wird eine Probe von 1 ml Kulturbrühe dem Kulturkolben entnommen.

### Fermentation und Aufreinigung

Für die Hauptkultur kommt ebenfalls ein Mineralmedium (M9) zum Einsatz. Die Fermentation nach Animpfen mit 10 vol. % Vorkultur und Verbrauch der vorgelegten Glucose erfolgt kohlenstofflimitiert über eine Glukosezufütterung in einem 2 Liter Fermenter mit einem Arbeitsvolumen von 1,2 L. Die Glukosezufütterung erfolgt anhand des Gelöstsauerstoffsignals. Der Gelöstsauerstoff wird bei 20 % Sättigung über die Rührerdrehzahl reguliert. Der pH Wert wird über eine pH Elektrode und Zugabe von NH₄SO₄ auf 7 reguliert. Die Fermentation wird über 4 Tage bis zu einer Biotrockenmasse von 15 g/l geführt. Die Rhamnolipidkonzentration wird über HPLC ermittelt und beträgt 9,8 g/l. Nach Abtrennen der Zellen mittels Zentrifugation bei 10.000 g wird die Fermentationsbrühe durch Zugabe konzentrierter HCl auf einen pH-Wert von 4,0 eingestellt. Anschließend wird mit gleichem Volumen Ethylacetat extrahiert. Die rhamnolipidhaltige organische Phase wird abgetrennt und weiter verarbeitet. Durch Zugabe von 50 Gew %iger KOH (aq) wird der pH-Wert der Lösung auf pH 7 eingestellt. Hierbei kommt es zur Bildung zweier flüssiger Phasen. Die untere Phase enthält die von lipophilen und hydrophilen Verunreinigungen befreiten Rhamnolipide in hoher Reinheit. Die Zusammensetzung der RL-Mischung wird hierdurch nicht beeinflusst. Die untere Phase wird abgenommen und das Lösungsmittel am Rotationsverdampfer weitestgehend entfernt. Dann wird erneut Wasser zugegeben und die wässrige RL Lösung gefriergetrocknet. Das erhaltene Pulver wird mittels HPLC analysiert und anwendungstechnisch charakterisiert.

### Quantifizierung von Rhamnolipiden

Die Probenvorbereitung für die nachfolgenden chromatographischen Analysen erfolgt folgendermaßen Mit einer Verdrängerpipette (Combitip) wird in einem 2 ml-Reaktionsgefäß 1 ml Aceton vorgelegt und das Reaktionsgefäß zur Minimierung von Verdunstung unmittelbar verschlossen. Es folgt die Zugabe von 1 ml Kulturbrühe. Nach Vortexen des Kulturbrühe/Acetongemisches wird dieses für 3 min bei 13.000 rpm abzentrifugiert, und 800 µl des Überstands in ein HPLC-Gefäß überführt.

Zum Nachweis und zur Quantifizierung von Rhamnolipiden wird ein Evaporative Light Scattering Detektor (Sedex LT-ELSD Model 85LT) benutzt. Die eigentliche Messung wird mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen beträgt 5 µl und die Laufzeit der Methode 20 min. Als mobile Phase wird wässrige 0,1 % TFA (Trifluor-Essigsäure, Lösung A) und Methanol (Lösung B) verwendet. Die Säulentemperatur beträgt 40 °C. Als Detektoren dienen der ELSD (Detektortemperatur 60 °C) und der DAD (Diodenarray, 210 nm). Der in der Methode verwendete Gradient ist:

| **t [min]** | **Lösung B vol.-%** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

Die mit oben beschriebenem Verfahren erhaltene Rhamnolipidzusammensetzung aus *P. putida* KT2440 pBBR1MCS2-Plac-rhIABC-T-Ptac-rhIC-T enthält:

| | |
|---|---|
| diRL-C10C10 | 81 Gew.-% |
| diRL-C10C12 | 10 Gew.-% |
| diRL-C10C12:1 | 8 Gew.-% |
| monoRL-C10C10 | 1 Gew.-% |

woraus ein Gewichtsverhältnis von di-Rhamnolipiden zu mono-Rhamnolipiden von 99:1 resultiert.

### Beispiel 4: Austestung der Hautpflegeleistung und Schaumeigenschaften mittels eines Handwaschtests

Zur Bewertung der Hautpflegeleistung und der Schaumeigenschaften von erfindungsgemäßen Produktbeispielen 2 und 3 in wässrigen, tensidischen Zusammensetzungen (Tensidformulierungen) wurden sensorische Handwaschtests im Vergleich zu bekannten Rhamnolipid-Zusammensetzungen durchgeführt.

Eine Gruppe bestehend aus 10 trainierten Prüfpersonen wusch sich bei diesem Handwaschtest definiert die Hände und bewertete Schaumeigenschaften und Hautgefühl anhand einer Notenskala von 1 (schlecht) bis 5 (sehr gut). Die eingesetzten Produkte wurden jeweils in einer standardisierten Tensidformulierung (Tabelle 1) getestet.

Als Kontrollformulierung A wurde eine Tensidformulierung ohne Zusatz eines Sekundärtensids verwendet. Die Tensidformulierungen B und C stellen die nichterfindungsgemäßen Vergleichsprodukte und die Tensidformulierungen D und E die erfindungsgemäße Zusammensetzung dar (Tabelle 1).

**Tabelle 1: Testformulierungen für Handwaschtest (Angaben in Gew.-%).**

| **Formulierungs-Beispiele** | **A** | **B** | **C** | **D** | **E** |
|---|---|---|---|---|---|
| Texapon® NSO (BASF Cognis, INCI: Sodium Laureth Sulfate, 28%-ig) | 32,1 | 32,1 | 32,1 | 32,1 | 32,1 |
| Beispiel 1: reines diRL-C10C10 (nicht erfindungsgemäß) | | 3,0 | | | |
| Jeneil Produkt (nicht erfindungsgemäß) | | | 3,0 | | |
| Beispiel 2 (erfindungsgemäß) | | | | 3,0 | |
| Beispiel 3 Fermentationsprodukt (erfindungsgemäß) | | | | | 3,0 |
| NaCl | 4,0 | 4,0 | 4,0 | 4,0 | 4,0 |
| Citric Acid, 30% | ad. pH 6,0 | | | | |
| Wasser, demineralisiert | ad. 100% | | | | |

In Tabelle 2 sind die Ergebnisse des Handwaschtests dargestellt.

**Tabelle 2: Ergebnisse des Handwaschtests**

| **Testformulierung** | **A** | **B** | **C** | **D** | **E** |
|---|---|---|---|---|---|
| Anschäumverhalten | 2,7 | 3,2 | 2,6 | 3,4 | 3,5 |
| Schaumvolumen | 2,6 | 2,7 | 2,3 | 2,7 | 2,7 |
| Schaumcremigkeit | 2,1 | 2,7 | 2,4 | 2,9 | 3,0 |
| Hautgefühl während des Waschens | 2,9 | 3,2 | 3,0 | 3,4 | 3,4 |
| Leichtigkeit des Abwaschens | 3,8 | 3,5 | 3,1 | 3,4 | 3,4 |
| Hautgefühl direkt nach dem Abwaschen | 2,4 | 2,0 | 2,2 | 2,3 | 2,3 |
| Hautglätte nach 3 min. | 2,8 | 3,3 | 3,1 | 3,5 | 3,7 |
| Hautweichheit nach 3 min. | 2,9 | 3,4 | 3,1 | 3,5 | 3,7 |

Anhand der Messergebnisse in Tabelle 2 wird ersichtlich, dass die erfindungsgemäßen Zusammensetzungen D und E unter Verwendung von Produktbeispiel 2 und 3 ein besseres Hautgefühl während des Waschens mit der Formulierung und überraschenderweise auch ein besseres Anschäumverhalten und erhöhte Schaumcremigkeit im Vergleich zur Kontrolle A und den Vergleichszusammensetzungen B und C nach dem Stand der Technik aufweisen. Zudem wird ersichtlich, dass die erfindungsgemäßen Zusammensetzungen D und E beim Hautgefühl (Hautglätte & Hautweichheit) nach dem Abwaschen und Trocknen besser bewertet wurden als die Vergleichsformulierungen.

Wider Erwarten zeigte in den vorliegenden Formulierungen ein bestimmter geringer Anteil an Monorhamnolipid im Biotensid einen positiven Einfluß auf das Schaumverhalten und das Hautgefühl.

### Beispiel 5: Austestung der Schaumeigenschaften mittels SITA Foam Tester

Die Anschäumbarkeit von Tensidlösungen ist eine wichtige Größe. Hieraus können Anwendungseigenschaften abgeleitet werden. Eine schnelle Schaumbildung und ein großes Schaumvolumen werden in vielen Anwendungen von guten Tensiden erwartet. Eine Methode um diese Größe zu bewerten basiert auf einem Schaumtestgerät SITA Foam Tester R-2000 der Firma SITA Messtechnik GmbH. Hierbei wird in ein definiertes Volumen einer Tensidlösung Luft durch eine Dispergierscheibe eingetragen und das Gesamtvolumen aus Flüssigkeit und entstehendem Schaum über die Zeit mittels Schaumsonden gemessen.
In einem solchen Gerät wurden drei verschiedene Rhamnolipidpräparationen bei pH = 6 und einer Rhamnolipidgesamtkonzentration von 0,5 Gew. % vermessen. Die Zusammensetzung der Rhamnolipidfraktionen ist in der folgenden Tabelle zusammengestellt.

| | E09-S6 | diRL-C10C10 | Jeneil |
|---|---|---|---|
| diRL-C8C10 | 21 Gew.-% | 0 Gew.-% | 2,1 Gew.-% |
| monoRL-C8C10 | 0,9 Gew.-% | 0 Gew.-% | 1,9 Gew.-% |
| diRL-C10C10 | 65 Gew.-% | 100 Gew.-% | 46,2 Gew.-% |
| monoRL-C10C10 | 1,6 Gew.-% | 0 Gew.-% | 29,8 Gew.-% |
| diRL-C10C12 | 6,0 Gew.-% | 0 Gew.-% | 7,8 Gew.-% |
| monoRL-C10C12 | 0 Gew.-% | 0 Gew.-% | 5,6 Gew.-% |
| diRL-C10C12:1 | 5,6 Gew.-% | 0 Gew.-% | 3,1 Gew.-% |
| monoRL-C10C12:1 | 0 Gew.-% | 0 Gew.-% | 3,1 Gew.-% |

Eine erfindungsgemäße Zusammensetzung E09-S6, die fermentativ gewonnen wurde, reine diRL-C10C10 und ein kommerziell verfügbares Produkt von Jeneil wurden untersucht.

Die Messungen zur Anschäumbarkeit wurden bei einer Temperatur von 30 °C mit einem Flüssigkeitsvolumen von 300 ml und eine Rührerdrehzahl von 1500 rpm durchgeführt.
Die Abbildung 1 zeigt, dass die Anschäumbarkeit von E09 S6 deutlich besser war als die der anderen Proben, das heißt das maximale Schaumvolumen wurde schneller erreicht.

## Patentansprüche

1. Mischungszusammensetzung enthaltend Rhamnolipide, **dadurch gekennzeichnet, dass** die Mischungszusammensetzung
51 Gew.-% bis 95 Gew.-% diRL-C10C10 und
0,5 Gew.-% bis 9 Gew.-% monoRL-C10C10
enthält,
wobei sich die Gewichtsprozente auf die Summe aller enthaltenen Rhamnolipide beziehen,
mit der Maßgabe, dass das Gewichtsverhältnis von di-Rhamnolipiden zu mono-Rhamnolipiden größer 91:9, bevorzugt größer 97:3, besonders bevorzugt größer 98:2 ist.

2. Mischungszusammensetzung gemäß Anspruch 1, **dadurch gekennzeichnet, dass** die Mischungszusammensetzung
0,5 Gew.-% bis 15 Gew.-% diRL-C10C12:1
enthält,
wobei sich die Gewichtsprozente auf die Summe aller enthaltenen Rhamnolipide beziehen.

3. Mischungszusammensetzung gemäß Anspruch 1 oder 2, **dadurch gekennzeichnet, dass** die Mischungszusammensetzung
0,5 bis 25 Gew.-% diRL-C10C12
enthält,
wobei sich die Gewichtsprozente auf die Summe aller enthaltenen Rhamnolipide beziehen.

4. Mischungszusammensetzung gemäß mindestens einem der vorherigen Ansprüche, **dadurch gekennzeichnet, dass** die Mischungszusammensetzung
0,1 Gew.-% bis 5 Gew.-% monoRL-C10C12 und/oder, bevorzugt und
0,1 Gew.-% bis 5 Gew.-% monoRL-C10C12:1,
enthält,
wobei sich die Gewichtsprozente auf die Summe aller enthaltenen Rhamnolipide beziehen.

5. Mischungszusammensetzung gemäß Anspruch 1, **dadurch gekennzeichnet, dass** sie
0,5 Gew.-% bis 15 Gew.-% diRL-C10C12:1,
0,5 bis 25 Gew.-% diRL-C10C12,
0,1 Gew.-% bis 5 Gew.-% monoRL-C10C12 und
0,1 Gew.-% bis 5 Gew.-% monoRL-C10C12:1,
enthält,
wobei sich die Gewichtsprozente auf die Summe aller enthaltenen Rhamnolipide beziehen.

6. Mischungszusammensetzung gemäß mindestens einem der vorherigen Ansprüche, **dadurch gekennzeichnet, dass** die Mischungszusammensetzung
0 Gew.-% bis 5 Gew.-% diRLC10
enthält,
wobei sich die Gewichtsprozente auf die Summe aller enthaltenen Rhamnolipide beziehen.

7. Mischungszusammensetzung gemäß mindestens einem der vorherigen Ansprüche, **dadurch gekennzeichnet, dass** die Mischungszusammensetzung mindestens 60 Gew.-%, bevorzugt mindestens 80 Gew.-%, besonders bevorzugt mindestens 90 Gew.-% Rhamnolipide enthält, wobei sich die Gewichtsprozente auf die Trockensubstanz der gesamten Mischungszusammensetzung beziehen.

8. Verfahren umfassend die Verfahrensschritte:
la) Bereitstellen einer *Pseudomonas putida* Zelle, die derart gentechnisch verändert wurde, dass sie jeweils mindestens ein Gen der Gruppe rhIA, rhIB und rhIC überexprimiert, wobei die Gene ausgewählt sind aus denen aus *P. aeruginosa,*
IIa) in Kontakt Bringen der erfindungsgemäßen Zelle mit einem Medium beinhaltend eine Kohlenstoffquelle
IIIa) Kultivieren der Zelle unter Bedingungen, die es der Zelle ermöglichen, aus der Kohlenstoffquelle Rhamnolipid zu bilden und
IVa) gegebenenfalls Isolierung der gebildeten Rhamnolipide,
**dadurch gekennzeichnet, dass** das Gen rhIC verglichen zu rhIB stärker überexprimiert wird.

9. Verfahren umfassend die Verfahrensschritte:
Ib) Bereitstellen einer *Pseudomonas putida* Zelle, die derart gentechnisch verändert wurde, dass sie jeweils mindestens ein exogenes Gen der Gruppe rhIA, rhIB und rhIC, von denen mindestens eines unter der Kontrolle eines induzierbaren Promotors steht, aufweist,
IIb) in Kontakt Bringen und Kultivieren der erfindungsgemäßen Zelle mit einem Medium beinhaltend mindestens eine Kohlenstoffquelle unter Erreichung einer Zelldichte von 1-30 g Zelltrockenmasse pro L Fermentationsbrühe,
IIIb) Induzieren des mindestens einen induzierbaren Promotors und Kultivieren der Zelle unter Bedingungen, die es der Zelle ermöglichen, aus der Kohlenstoffquelle Rhamnolipid zu bilden und
IVb) gegebenenfalls Isolierung der gebildeten Rhamnolipide.

10. Verwendung einer Mischungszusammensetzung gemäß mindestens einem der Ansprüche 1 bis 7 zur Herstellung von Formulierungen.

11. Verwendung einer Mischungszusammensetzung gemäß mindestens einem der Ansprüche 1 bis 7 zur Reinigung einer Oberfläche.

## Claims

1. Mixture composition comprising rhamnolipids, **characterized in that** the mixture composition comprises
51% by weight to 95% by weight of diRL-C10C10 and 0.5% by weight to 9% by weight of monoRL-C10C10, where the percentages by weight refer to the sum of all of the rhamnolipids present,
with the proviso that the weight ratio of di-rhamnolipids to mono-rhamnolipids is greater than 91:9, preferably greater than 97:3, particularly preferably greater than 98:2.

2. Mixture composition according to Claim 1, **characterized in that** the mixture composition comprises
0.5% by weight to 15% by weight of diRL-C10C12:1, where the percentages by weight refer to the sum of all of the rhamnolipids present.

3. Mixture composition according to Claim 1 or 2, **characterized in that** the mixture composition comprises
0.5 to 25% by weight of diRL-C10C12,
where the percentages by weight refer to the sum of all of the rhamnolipids present.

4. Mixture composition according to at least one of the preceding claims, **characterized in that** the mixture composition comprises
0.1% by weight to 5% by weight of monoRL-C10C12 and/or,
preferably and
0.1% by weight to 5% by weight of monoRL-C10C12:1,
where the percentages by weight refer to the sum of all of the rhamnolipids present.

5. Mixture composition according to Claim 1, **characterized in that** it comprises
0.5% by weight to 15% by weight of diRL-C10C12:1,
0.5 to 25% by weight of diRL-C10C12,
0.1% by weight to 5% by weight of monoRL-C10C12 and
0.1% by weight to 5% by weight of monoRL-C10C12:1,
where the percentages by weight refer to the sum of all of the rhamnolipids present.

6. Mixture composition according to at least one of the preceding claims, **characterized in that** the mixture composition comprises
0% by weight to 5% by weight of diRLC10,
where the percentages by weight refer to the sum of all of the rhamnolipids present.

7. Mixture composition according to at least one of the preceding claims, **characterized in that** the mixture composition comprises at least 60% by weight, preferably at least 80% by weight, particularly preferably at least 90% by weight, of rhamnolipids, where the percentages by weight refer to the dry substance of the overall mixture composition.

8. Process comprising the process steps:
Ia) providing a *Pseudomonas putida* cell which has been genetically modified in such a way that it overexpresses in each case at least one gene of the group rhlA, rhlB and rhlC, wherein the genes selected are from those from *P*. *aeruginosa*,
IIa) bringing the cell according to the invention into contact with a medium comprising a carbon source,
IIIa) cultivating the cell under conditions which allow the cell to form rhamnolipid from the carbon source and
IVa) optionally isolating the rhamnolipids formed, **characterized in that** the gene rhlC is overexpressed more compared to rhlB.

9. Process comprising the process steps:
Ib) providing a *Pseudomonas putida* cell which has been genetically modified in such a way that it has in each case at least one exogenous gene of the group rhlA, rhlB and rhlC, of which at least one is under the control of an inducible promoter,
IIb) bringing the cell according to the invention into contact with, and cultivating it with a medium comprising at least one carbon source while achieving a cell density of 1-30 g of cell dry mass per L of fermentation broth,
IIIb) inducing the at least one inducible promoter and cultivating the cell under conditions which allow the cell to form rhamnolipid from the carbon source and
IVb) optionally isolating the rhamnolipids formed.

10. Use of a mixture composition according to at least one Claims 1 to 7 for producing formulations.

11. Use of a mixture composition according to at least one Claims 1 to 7 for cleaning a surface.

## Revendications

1. Composition de mélange contenant des rhamnolipides, **caractérisée en ce que** la composition de mélange contient :
51 % en poids à 95 % en poids de diRL-C10C10 et
0,5 % en poids à 9 % en poids de monoRL-C10C10,
les pourcentages en poids se rapportant à la somme de tous les rhamnolipides contenus,
à condition que le rapport en poids entre les di-rhamnolipides et les mono-rhamnolipides soit supérieur à 91:9, de préférence supérieur à 97:3, de manière particulièrement préférée supérieur à 98:2.

2. Composition de mélange selon la revendication 1, **caractérisée en ce que** la composition de mélange contient :
0,5 % en poids à 15 % en poids de diRL-C10C12:1,
les pourcentages en poids se rapportant à la somme de tous les rhamnolipides contenus.

3. Composition de mélange selon la revendication 1 ou 2, **caractérisée en ce que** la composition de mélange contient :
0,5 à 25 % en poids de diRL-C10C12,
les pourcentages en poids se rapportant à la somme de tous les rhamnolipides contenus.

4. Composition de mélange selon au moins l'une quelconque des revendications précédentes, **caractérisée en ce que** la composition de mélange contient :
0,1 % en poids à 5 % en poids de monoRL-C10C12 et/ou, de préférence et,
0,1 % en poids à 5 % en poids de monoRL-C10C12:1,
les pourcentages en poids se rapportant à la somme de tous les rhamnolipides contenus.

5. Composition de mélange selon la revendication 1, **caractérisée en ce qu'**elle contient :
0,5 % en poids à 15 % en poids de diRL-C10C12:1,
0,5 à 25 % en poids de diRL-C10C12,
0,1 % en poids à 5 % en poids de monoRL-C10C12 et
0,1 % en poids à 5 % en poids de monoRL-C10C12:1,
les pourcentages en poids se rapportant à la somme de tous les rhamnolipides contenus.

6. Composition de mélange selon au moins l'une quelconque des revendications précédentes, **caractérisée en ce que** la composition de mélange contient :
0 % en poids à 5 % en poids de diRLC10,
les pourcentages en poids se rapportant à la somme de tous les rhamnolipides contenus.

7. Composition de mélange selon au moins l'une quelconque des revendications précédentes, **caractérisée en ce que** la composition de mélange contient au moins 60 % en poids, de préférence au moins 80 % en poids, de manière particulièrement préférée au moins 90 % en poids de rhamnolipides, les pourcentages en poids se rapportant à la substance sèche de la composition de mélange totale.

8. Procédé comprenant les étapes de procédé suivantes :
Ia) la préparation d'une cellule de *Pseudomonas putida*, qui a été modifiée génétiquement de telle sorte qu'elle surexprime au moins un gène du groupe constitué par rhIA, rhIB et rhIC, les gènes étant choisis parmi ceux issus de *P. aeruginosa,*
IIa) la mise en contact de la cellule selon l'invention avec un milieu contenant une source de carbone,
IIIa) la culture de la cellule dans des conditions qui permettent à la cellule de former un rhamnolipide à partir de la source de carbone, et
IVa) éventuellement l'isolement des rhamnolipides formés,
**caractérisé en ce que** le gène rhIC est plus fortement surexprimé en comparaison de rhIB.

9. Procédé comprenant les étapes de procédé suivantes :
Ib) la préparation d'une cellule de *Pseudomonas putida*, qui a été modifiée génétiquement de telle sorte qu'elle comprenne au moins un gène exogène du groupe constitué par rhIA, rhIB et rhIC, parmi lesquels au moins un se trouve sous le contrôle d'un promoteur inductible,
IIb) la mise en contact et la culture de la cellule selon l'invention avec un milieu contenant au moins une source de carbone pour obtenir une densité cellulaire de 1 à 30 g de masse cellulaire sèche par 1 de bouillon de fermentation,
IIIb) l'induction dudit au moins un promoteur inductible et la culture de la cellule dans des conditions qui permettent à la cellule de former un rhamnolipide à partir de la source de carbone, et
IVb) éventuellement l'isolement des rhamnolipides formés.

10. Utilisation d'une composition de mélange selon au moins l'une quelconque des revendications 1 à 7 pour la fabrication de formulations.

11. Utilisation d'une composition de mélange selon au moins l'une quelconque des revendications 1 à 7 pour le nettoyage d'une surface.
